# EUROPEAN PATENT SPECIFICATION

(11) **EP 3 316 942 B1**
(45) Date of publication and mention of the grant of the patent: **26.02.2020**
(21) Application number: 16733978.7
(22) Date of filing: 01.07.2016
(51) Int. Cl.: A61M 5/31, A61M 5/315, A61M 5/24

(54) **DRUG DELIVERY DEVICE WITH PRIMING MECHANISM**
WIRKSTOFFFREISETZUNGSVORRICHTUNG MIT SPÜLMECHANISMUS
DISPOSITIF DE DISTRIBUTION DE MÉDICAMENTS À MÉCANISME D'AMORÇAGE

(30) Priority: 01.07.2015 EP 15174923
(43) Date of publication of application: 09.05.2018
(73) Proprietor: Novo Nordisk A/S, 2880 Bagsværd (DK)
(72) Inventor: BENGTSSON, Henrik, 2880 Bagsværd (DK); NØRBYGAARD, Anders, 2880 Bagsværd (DK); CARROLL, Ronan, 2880 Bagsværd (DK); SPORK, Emil Gram, 2200 Copenhagen N (DK)
(86) International application number: PCT/EP2016/065619
(87) International publication number: WO 2017/001692

(56) References cited:
- WO-A1-2009/095332
- WO-A1-2011/113868
- US-A1- 2010 280 461
- US-A1- 2011 245 780

## Description

The present invention relates to an assembly of components for a drug delivery device that allows a user to select single or multiple doses of an injectable liquid drug and to dispense the set dose of the product and to apply said product to a patient, preferably by injection. In particular, the present invention relates to a method of assembling such drug delivery devices.

### BACKGROUND

In the disclosure of the present invention reference is mostly made to drug delivery devices used e.g. in the treatment of diabetes by delivery of insulin, however, this is only an exemplary use of the present invention.

Drug delivery devices allowing for multiple dosing of a required dosage of a liquid medicinal product, such as liquid drugs, and further providing administration of the liquid to a patient, are as such well-known in the art. Generally, such devices have substantially the same purpose as that of an ordinary syringe. Drug delivery devices of this kind have to meet a number of user specific requirements. For instance in case of those with diabetes, many users will be physically infirm and may also have impaired vision. Therefore, these devices need to be robust in construction, yet easy to use, both in terms of the manipulation of the parts and understanding by a user of its operation. Further, the dose setting must be easy and unambiguous and where the device is to be disposable rather than reusable, the device should be inexpensive to manufacture and easy to dispose. In order to meet these requirements, the number of parts and steps required to assemble the device and an overall number of material types the device is made from have to be kept to a minimum.

Typically, the liquid drug to be administered is provided in a cartridge that has a moveable piston or bung mechanically interacting with a piston rod of an expelling mechanism of the drug delivery device. By applying thrust to the piston in distal direction, a predefined amount of the liquid drug is expelled from the cartridge. Due to inevitable manufacturing tolerances there may for instance persist axial clearance between a cartridge's piston and the piston rod. Typically, prior to a primary use of the device, an end-user has to conduct a so-called priming of the expelling mechanism in order to ensure, that already with an initial dose setting and a first subsequent dose dispensing step, an accurate amount of the liquid drug is dispensed in a predefined way.

An initial dose setting and expelling of a minor dose may in certain situations also be required for removing any air present in the cartridge and/or a connected needle and for performing a flow check.

Document WO 99/38554 A1 discloses several embodiments of injection devices each suitable for forming a disposable device wherein a liquid drug cartridge is inserted into the device during assembly in a production line.

State of the art pen-type drug delivery devices that incorporate a dose setting feature often include a so-called end-of-content limiter to prevent a user from selecting a size of a dose which exceeds the amount of liquid drug remaining in a cartridge of the device. References WO 01/19434 A1, WO 2006/128794 A2 and WO 2010/149209 A1, WO 2007/017052 A1 and WO 2013/156224 A1 include disclosure of such end-of-content limiters.

In the production line, during final assembly operations of the devices, at least a part of the priming is typically carried out using the dose setting and expelling mechanism so that users will experience virtually consistent requirement for a priming operation across individual pen samples irrespective of the initial gap between the piston rod and the piston which emanates from tolerances. Reference WO 2009/095332 A1 discloses devices wherein a distance between the distal end of a piston rod means and the plunger is minimized or reduced to zero.

### SUMMARY

It is an object of the present invention to provide a drug delivery device featuring improved and facilitated clearance reduction or clearance elimination. It is a further object of the invention to provide a simplified and robust method of eliminating clearance in a drug delivery device. Finally, it is an object of the invention to provide manufacture of drug delivery devices providing consistently uniform and predictable total doseable amount of liquid drug from a held cartridge.

In the disclosure of the present invention, embodiments and aspects will be described which will address one or more of the above objects or which will address objects apparent from the below disclosure as well as from the description of exemplary embodiments.

The scope of the invention is defined by the independent claim. Further embodiments are described in the dependent claims.

The present invention relates to a drug delivery device for expelling a dose of drug from a held cartridge, the drug delivery device defining a distal drug outlet end and an opposite proximal end and comprising:
- a distal housing component holding a cartridge comprising a liquid drug and a piston slideable arranged therein in an axial direction,
- a proximal housing component housing at least in part a dose setting and expelling mechanism comprising a piston rod for exerting a force on the piston of cartridge in a distal direction for expelling a dose,
wherein the distal housing component and the proximal housing component are displaceably arranged in axial direction relative to each other for reducing an axial distance between the piston rod and the piston of the cartridge,
wherein a first geometry of the distal housing component cooperates with a second geometry of the proximal housing component to enable relative axial displacement but prevent relative rotation,
wherein one of the distal housing component and the proximal housing component comprises a threaded adjustment component being held at an axial fixed location on said one of the distal housing component and the proximal housing component and defining a first thread being in threaded engagement with a second thread defined by the other of the distal housing component and the proximal housing component,
and
wherein the threaded adjustment component is rotatably fixated so that the adjustment component cannot be rotated without the use of a tool.

In accordance with the first aspect the invention provides an effective means of adjusting or eliminating clearance between the piston rod and the piston of a held cartridge during assembly operations. By a simple adjustment procedure, the axial position between the distal housing component and the proximal housing component is adjusted and thus an initial clearance may be reduced into a predefined magnitude or alternatively eliminated completely.

In some embodiments, the threaded adjustment component is axially held at an axial fixed position relative to the proximal housing component and the distal housing component defines said second thread.

In other embodiments, the threaded adjustment component is axially held at an axial fixed position relative to the distal housing and the proximal housing component defines said second thread.

In some embodiments, the dose setting and expelling mechanism comprises a dose setting element and a driver configured to drive the piston rod in the distal direction.

The dose setting and expelling mechanism may comprise a dose setting element that rotates relative to the driver during setting of a dose and wherein the dose setting element and the driver rotates together during expelling of a set dose.

In particular embodiments of the invention the drug delivery device comprises an end-of-content limiter which prevents setting a dose which exceeds a doseable amount of liquid drug remaining in the cartridge. When using the adjustment procedure according to the first aspect of the invention, the adjustment procedure may be utilized to ensure that the total doseable volume that is expellable by each device is constant or near constant even though tolerances of the different parts of the device potentially would potentially cause the total doseable volume to vary from one device to another.

In some embodiments, the end-of-content limiter may some embodiments be arranged between the piston rod and the dose setting element of the drug delivery device. In some forms, the end-of-content limiter is engaging the piston rod and a dose setting element or an element that is associated with the dose setting element. In such embodiments, as the dose setting element is rotated relative to the piston rod for dialling up a dose, the end-of-content limiter moves towards an end-of-content stop geometry. The end-of-content stop geometry may for example be provided by one of the piston rod and the dose setting element.

The end-of-content limiter may in other embodiments be arranged between the driver and the dose setting element of the drug delivery device. In exemplary embodiments the end-of-content limiter is engaging the driver and the dose setting element. In such embodiment, as the dose setting element is rotated relative to the driver for dialling up a dose, the end-of-content limiter moves towards an end-of-content stop geometry for example provided by one of the driver and the dose setting element.

As used herein, the term "insulin" is meant to encompass any drug-containing flowable medicine capable of being passed through a delivery means such as a cannula or hollow needle in a controlled manner, such as a liquid, solution, gel or fine suspension, and which has a blood glucose controlling effect, e.g. human insulin and analogues thereof as well as non-insulins such as GLP-1 and analogues thereof. In the description of exemplary embodiments reference will be made to the use of insulin.

### BRIEF DESCRIPTION OF DRAWINGS

In the following the invention will be further described with reference to the drawings, wherein
fig. 1 shows a perspective view of a prior art pen device,
fig. 2 shows in an exploded view the components of the pen device of fig. 1,
figs. 3A and 3B show in sectional views an expelling mechanism of the pen device of fig. 1 in two states,
figs. 3C - 3E show components of the pen device of fig. 1,
fig. 4 is a perspective view of main components for a pen device 100 according to the invention,
fig. 5A through 5F show different assembly states during assembly of pen device 100.

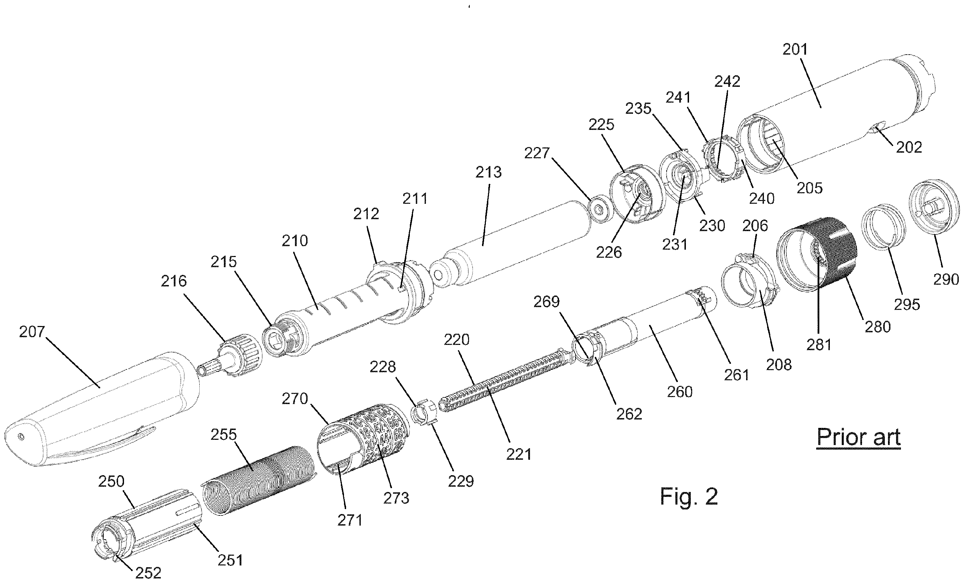

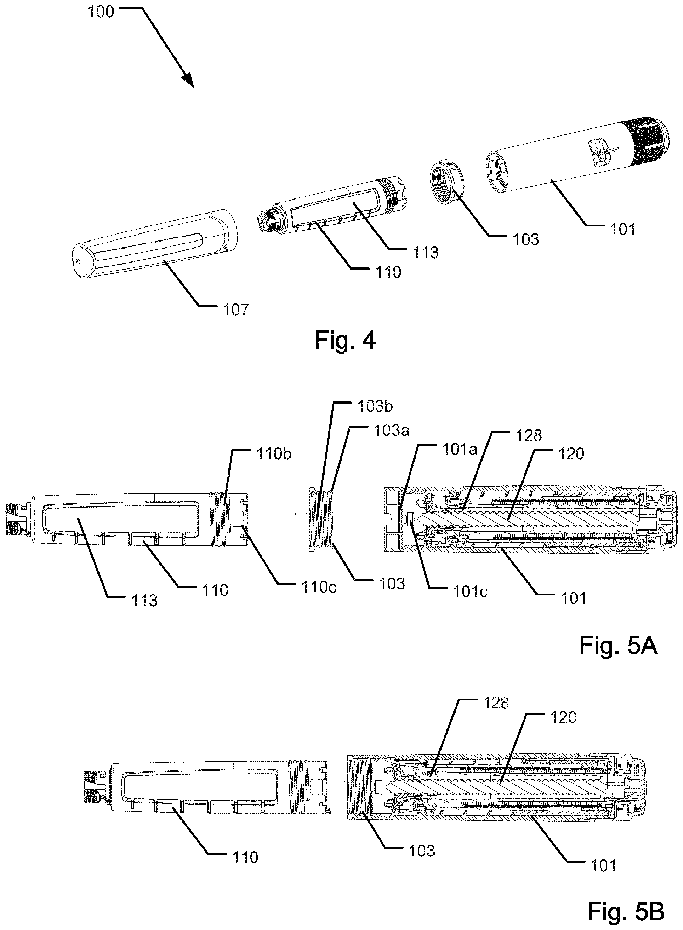

Generally, in the figures, like structures are mainly identified by like reference numerals, e.g. so that parts carrying reference no. "220" in fig. 2 correspond to reference no. "120" in figs. 4 through 5F.

### DESCRIPTION

When in the following terms such as "upper" and "lower", "right" and "left", "horizontal" and "vertical" or similar relative expressions are used, these only refer to the appended figures and not necessarily to an actual situation of use. The shown figures are schematic representations for which reason the configuration of the different structures as well as their relative dimensions are intended to serve illustrative purposes only. When the term member or element is used for a given component it generally indicates that in the described embodiment the component is a unitary component, however, the same member or element may alternatively comprise a number of sub-components just as two or more of the described components could be provided as unitary components, e.g. manufactured as a single injection moulded part. The term "assembly" does not imply that the described components necessarily can be assembled to provide a unitary or functional assembly during a given assembly procedure but is merely used to describe components grouped together as being functionally more closely related.

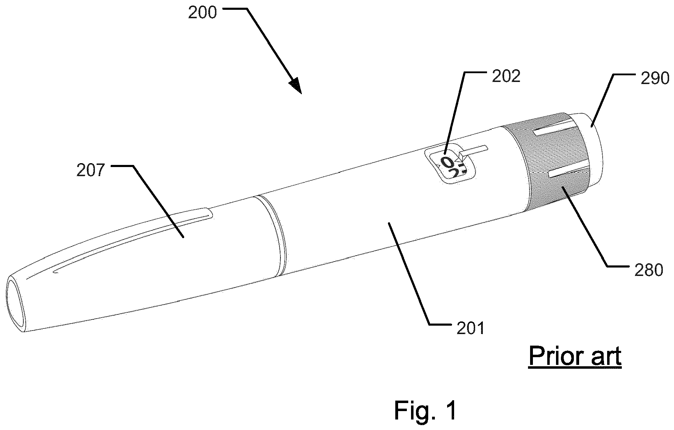

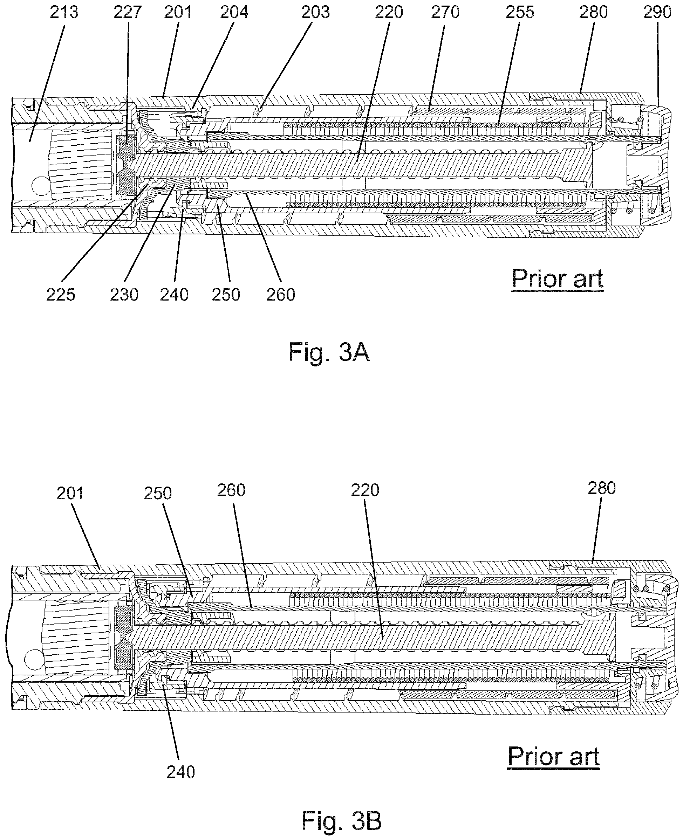

Figs. 1 shows a prior art drug delivery device in the form of a pen-formed auto-injection device 200, i.e. a so-called "injection pen" that includes an expelling mechanism incorporating a spring drive. Fig. 2 shows an exploded view of the prior art auto-injection device 200 shown in fig. 1. Figs. 3A and 3B show cross sectional views of the expelling mechanism of the prior art auto-injection device 200 shown in figs. 1 and 2 where fig. 3A shows the device in dose setting state and fig. 3B shows the device in dose expelling state.

Before turning to embodiments of the present invention per se, an example of a pre-filled drug delivery 200 will be described, such a device providing a potential basis for the exemplary embodiments of the present invention.

The prior art pen device 200 comprises a cap part 207 and a main part having a proximal body or drive assembly portion with a housing 201 in which a drug expelling mechanism is arranged or integrated, and a distal cartridge holder portion in which a drug-filled transparent cartridge 213 with a distal needle-penetrable septum is arranged and retained in place by a non-removable cartridge holder attached to the proximal portion, the cartridge holder having openings allowing a portion of the cartridge to be inspected as well as distal coupling means 215 allowing a needle assembly to be releasably mounted. The cartridge is provided with a piston driven by a piston rod forming part of the expelling mechanism and may for example contain an insulin, GLP-1 or growth hormone formulation. A proximal-most rotatable dose dial member 280 serves to manually set a desired dose of drug shown in display window 202 and which can then be expelled when the release button 290 is actuated. Depending on the type of expelling mechanism embodied in the drug delivery device, the expelling mechanism may comprise a spring as in the shown embodiment which is strained during dose setting and then released to drive the piston rod when the release button 290 is actuated.

As appears, fig. 1 shows a drug delivery device of the pre-filled type, i.e. it is supplied with a pre-mounted cartridge and is to be discarded when the cartridge has been emptied. In alternative embodiments, and in accordance with the present invention, the drug delivery device may be designed to allow a loaded cartridge to be replaced, e.g. in the form of a "rear-loaded" drug delivery device in which the cartridge holder is adapted to be removed from the device main portion, or alternatively in the form of a "front-loaded" device in which a cartridge is inserted through a distal opening in the cartridge holder which is non-removable attached to the main part of the device.

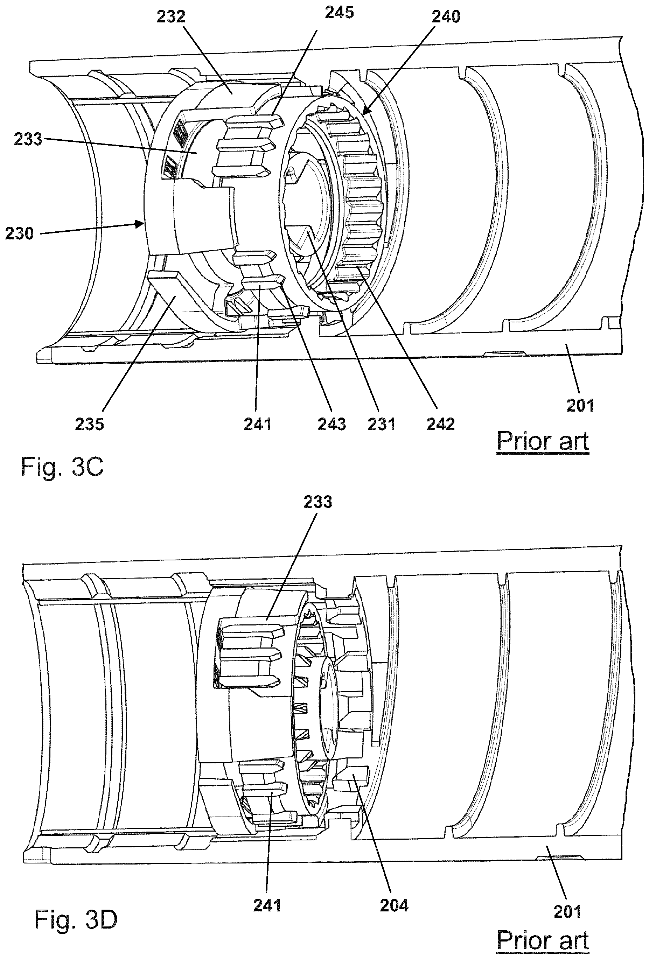

More specifically, referring to fig. 2, the pen comprises a tubular housing 201 with a window opening 202 and onto which a cartridge holder 210 is fixedly mounted, a drug-filled cartridge 213 being arranged in the cartridge holder. The cartridge holder is provided with distal coupling means 215 allowing a needle assembly 216 to be releasably mounted, proximal coupling means in the form of two opposed protrusions 211 allowing a cap 207 to be releasably mounted covering the cartridge holder and a mounted needle assembly, as well as a protrusion 212 preventing the pen from rolling on e.g. a table top. In the housing distal end a nut element 225 is fixedly mounted, the nut element comprising a central threaded bore 226, and in the housing proximal end a spring base member 208 with a central opening is fixedly mounted. A drive system comprises a threaded piston rod 220 having two opposed longitudinal grooves and being received in the nut element threaded bore, a ring-formed piston rod drive element 230 rotationally arranged in the housing, and a ring-formed clutch element 240 which is in rotational engagement with the drive element (see below), the engagement allowing axial movement of the clutch element. The clutch element is provided with outer spline elements 241 adapted to engage corresponding splines on the housing inner surface, this allowing the clutch element to be moved between a rotationally locked proximal position, in which the splines are in engagement, and a rotationally free distal position in which the splines are out of engagement. As just mentioned, in both positions the clutch element 240 is rotationally locked to the drive element 230. The drive element comprises a central bore with two opposed protrusions 231 in engagement with the grooves on the piston rod whereby rotation of the drive element results in rotation and thereby distal axial movement of the piston rod due to the threaded engagement between the piston rod and the nut element. The drive element further comprises a pair of opposed circumferentially extending flexible ratchet arms 235 adapted to engage corresponding ratchet teeth 205 arranged on the housing inner surface. The drive element and the clutch element comprise cooperating coupling structures rotationally locking them together but allowing the clutch element to be moved axially, this allowing the clutch element to be moved axially to its distal position in which it is allowed to rotate, thereby transmitting rotational movement from the dial system (see below) to the drive system. The interaction between the clutch element, the drive element and the housing will be shown and described in greater detail with reference to figs. 3C and 3D.

On the piston rod an end-of-content (EOC) member 228 (end-of-content limiter) is threadedly mounted and on the distal end a washer 227 is rotationally mounted. The EOC member comprises a pair of opposed radial projections 229 for engagement with the reset tube (see below).

The dial system comprises a ratchet tube 250, a reset tube 260, a scale drum 270 with an outer helically arranged row of dose numerals, a user-operated dose dial member 280 for setting a dose of drug to be expelled, a release button 290 and a torque spring 255 (see fig. 3A and 3B). The reset tube is mounted axially locked inside the ratchet tube but is allowed to rotate a few degrees (see below). The reset tube comprises on its inner surface two opposed longitudinal grooves 269 adapted to engage the radial projections 229 of the EOC member, whereby the EOC can be rotated by the reset tube but is allowed to move axially. The clutch element is mounted axially locked on the outer distal end portion of the ratchet tube 250, this providing that the ratchet tube can be moved axially in and out of rotational engagement with the housing via the clutch element. The dose dial member 280 is mounted axially locked but rotationally free on the housing proximal end, the dose dial member being under normal operation rotationally locked to the reset tube (see below), whereby rotation of dose dial member results in a corresponding rotation of the reset tube and thereby the ratchet tube. The release button 290 is axially locked to the reset tube but is free to rotate. A return spring 295 provides a proximally directed force on the button and the thereto mounted reset tube. The scale drum 270 is arranged in the circumferential space between the ratchet tube and the housing, the drum being rotationally locked to the ratchet tube via cooperating longitudinal splines 251, 271 and being in rotational threaded engagement with the inner surface of the housing via cooperating thread structures 203, 273, whereby the row of numerals passes the window opening 202 in the housing when the drum is rotated relative to the housing by the ratchet tube. The torque spring is arranged in the circumferential space between the ratchet tube and the reset tube and is at its proximal end secured to the spring base member 208 and at its distal end to the ratchet tube, whereby the spring is strained when the ratchet tube is rotated relative to the housing by rotation of the dial member. A ratchet mechanism with a flexible ratchet arm 252 is provided between the ratchet tube and the clutch element, the latter being provided with an inner circumferential teeth structures 242, each tooth providing a ratchet stop such that the ratchet tube is held in the position to which it is rotated by a user via the reset tube when a dose is set. In order to allow a set dose to be reduced a ratchet release mechanism 262 is provided on the reset tube and acting on the ratchet tube, this allowing a set dose to be reduced by one or more ratchet increments by turning the dial member in the opposite direction, the release mechanism being actuated when the reset tube is rotated the above-described few degrees relative to the ratchet tube.

Having described the different components of the expelling mechanism and their functional relationship, operation of the mechanism will be described next with reference mainly to figs. 3A and 3B.

The pen mechanism can be considered as two interacting systems, a dose system and a dial system, this as described above. During dose setting the dial mechanism rotates and a torsion spring of the spring drive is loaded. The dose mechanism is locked to the housing and cannot move. When the push button is pushed down, the dose mechanism is released from the housing and due to the engagement to the dial system, the torsion spring will now rotate back the dial system to the starting point and rotate the dose system along with it.

The central part of the dose mechanism is the piston rod 220, the actual displacement of the plunger being performed by the piston rod. During dose delivery, the piston rod is rotated by the drive element 230 and due to the threaded interaction with the nut element 225 which is fixed to the housing, the piston rod moves forward in the distal direction. Between the rubber piston and the piston rod, the piston washer 227 is placed which serves as an axial bearing for the rotating piston rod and evens out the pressure on the rubber piston. As the piston rod has a non-circular cross section where the piston rod drive element engages with the piston rod, the drive element is locked rotationally to the piston rod, but free to move along the piston rod axis. Consequently, rotation of the drive element results in a linear forwards movement of the piston. The drive element is provided with small ratchet arms 234 which prevent the drive element from rotating clockwise (seen from the push button end). Due to the engagement with the drive element, the piston rod can thus only move forwards. During dose delivery, the drive element rotates anti-clockwise and the ratchet arms 235 provide the user with small clicks due to the engagement with the ratchet teeth 205, e.g. one click per unit of insulin expelled.

Turning to the dial system, the dose is set and reset by turning the dose dial member 280. When turning the dial, the reset tube 260, the EOC member 228, the ratchet tube 250 and the scale drum 270 all turn with it. As the ratchet tube is connected to the distal end of the torque spring 255, the spring is loaded. During dose setting, the arm 252 of the ratchet performs a dial click for each unit dialed due to the interaction with the inner teeth structure 242 of the clutch element. In the shown embodiment the clutch element is provided with 24 ratchet stops providing 24 clicks (increments) for a full 360 degrees rotation relative to the housing. The spring is preloaded during assembly which enables the mechanism to deliver both small and large doses within an acceptable speed interval. As the scale drum is rotationally engaged with the ratchet tube, but movable in the axial direction and the scale drum is in threaded engagement with the housing, the scale drum will move in a helical pattern when the dial system is turned, the number corresponding to the set dose being shown in the housing window 202.

The ratchet 252, 242 between the ratchet tube and the clutch element 240 prevents the spring from turning back the parts. During resetting, the reset tube moves the ratchet arm 252, thereby releasing the ratchet click by click, one click corresponding to one unit IU of insulin in the described embodiment. More specifically, when the dial member is turned clockwise, the reset tube simply rotates the ratchet tube allowing the arm of the ratchet to freely interact with the teeth structures 242 in the clutch element. When the dial member is turned counter-clockwise, the reset tube interacts directly with the ratchet click arm forcing the click arm towards the centre of the pen away from the teeth in the clutch, thus allowing the click arm on the ratchet to move "one click" backwards due to torque caused by the loaded spring.

To deliver a set dose, the release button 290 is pushed in the distal direction by the user as shown in fig. 3B. The reset tube 260 decouples from the dial member and subsequently the clutch element 240 disengages the housing splines 204. Now the dial mechanism returns to "zero" together with the drive element 230, this leading to a dose of drug being expelled. It is possible to stop and start a dose at any time by releasing or pushing the push button at any time during drug delivery. A dose of less than 5 IU normally cannot be paused, since the rubber piston is compressed very quickly leading to a compression of the rubber piston and subsequently delivery of insulin when the piston returns to the original dimensions.

The EOC feature prevents the user from setting a larger dose than left in the cartridge. The EOC member 228 is rotationally locked to the reset tube, which makes the EOC member rotate during dose setting, resetting and dose delivery, during which it can be moved axially back and forth following the thread of the piston rod. When it reaches the proximal end of the piston rod a stop is provided, this preventing all the connected parts, including the dial member, from being rotated further in the dose setting direction, i.e. the now set dose corresponds to the remaining drug content in the cartridge.

The scale drum 270 is provided with a distal stop surface 274 adapted to engage a corresponding stop surface on the housing inner surface, this providing a maximum dose stop for the scale drum preventing all the connected parts, including the dial member, from being rotated further in the dose setting direction. In the shown embodiment the maximum dose is set to 80 IU. Correspondingly, the scale drum is provided with a proximal stop surface adapted to engage a corresponding stop surface on the spring base member, this preventing all the connected parts, including the dial member, from being rotated further in the dose expelling direction, thereby providing a "zero" stop for the entire expelling mechanism. In the following, the position that the dial member assumes after completion of the expelling of a set dose will be referred to as the "zero dose position".

To prevent accidental over-dosage in case something should fail in the dialing mechanism allowing the scale drum to move beyond its zero-position, the EOC member serves to provide a security system. More specifically, in an initial state with a full cartridge the EOC member is positioned in a distal-most axial position in contact with the drive element. After a given dose has been expelled the EOC member will again be positioned in contact with the drive element. Correspondingly, the EOC member will lock against the drive element in case the mechanism tries to deliver a dose beyond the zero-position. Due to tolerances and flexibility of the different parts of the mechanism the EOC will travel a short distance allowing a small "over dose" of drug to be expelled, e.g. 3-5 IU of insulin.

The expelling mechanism further comprises an end-of-dose (EOD) click feature providing a distinct feedback at the end of an expelled dose informing the user that the full amount of drug has been expelled. More specifically, the EOD function is made by the interaction between the spring base and the scale drum. When the scale drum returns to zero, a small click arm 206 on the spring base is forced backwards by the progressing scale drum. Just before "zero" the arm is released and the arm hits a countersunk surface on the scale drum.

The shown mechanism is further provided with a torque limiter in order to protect the mechanism from overload applied by the user via the dose dial member. This feature is provided by the interface between the dose dial member and the reset tube which as described above are rotationally locked to each other. More specifically, the dose dial member is provided with a circumferential inner teeth structure 281 engaging a number of corresponding teeth arranged on a flexible carrier portion 261 of the reset tube. The reset tube teeth are designed to transmit a torque of a given specified maximum size, e.g. 150-300 Nmm, above which the flexible carrier portion and the teeth will bend inwards and make the dose dial member turn without rotating the rest of the dial mechanism. Thus, the mechanism inside the pen cannot be stressed at a higher load than the torque limiter transmits through the teeth.

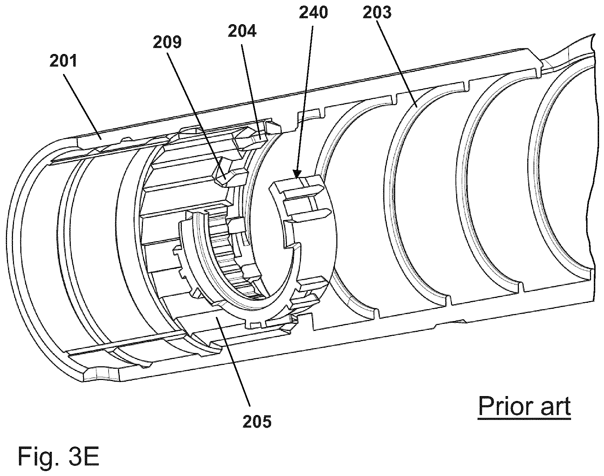

In fig. 3C the clutch element, the drive element and the housing (in partial) are shown in the dose setting state, and in fig. 3D the same components are shown in the expelling state. As appears, the piston rod on which the drive element is arranged and the ratchet tube on which the clutch element is mounted are not shown. To better show the structures provided on the inner surface of the housing fig. 3E shows a partial clutch element 240 arranged in the housing 201.

The inner surface of the housing 201 comprises a circumferential ring-formed array of axially oriented spline elements 204 protruding into the interior, each having a pointed distal end 209, as well as a circumferential ring-formed array of one-way ratchet teeth 205. The inner surface further comprises a male helical thread 203 adapted to engage the female helical thread 273 on the scale drum 270. A distal circumferential groove is formed to engage and mount the nut element 225. The clutch element 240 comprises an inner circumferential ring-formed array of ratchet teeth 242 adapted to engage the ratchet arm 252 on the ratchet tube 250, and an outer circumferential ring-formed array of axially oriented spline elements 241 adapted to engage the spline elements 204 of the housing as well as the coupling slots in the drive element (see below), each spline having a pointed proximal end 243. The drive element 230 comprises a pair of opposed coupling portions each comprising two proximally extending skirt portions 232 between which an axially extending coupling slot 233 is formed, the slot being adapted to engage a portion of the clutch element spline elements. In this way the engaging surfaces serve to transmit a rotational force and thereby torque from the clutch element to the drive element in the expelling state. The drive element further comprises a pair of opposed circumferentially extending flexible ratchet arms adapted to engage the ring-formed array of one-way ratchet teeth 205. During dose delivery, the drive element rotates anti-clockwise and the ratchet arms 235 also provide the user with small clicks due to the engagement with the ratchet teeth 205, e.g. one click per unit of insulin expelled. In the shown embodiment 24 ratchet teeth are provided corresponding to 15 degrees rotation per unit of insulin. The central bore of the drive element comprises two opposed protrusions 231 adapted to engage with the axially oriented grooves on the piston rod.

In the dose setting state shown in fig. 3C the spline elements 241 of the clutch element are in engagement with the spline elements 204 of the housing thereby rotationally locking the clutch element relative to the housing. As can be seen from fig. 3C a group of clutch spline elements are received in the corresponding coupling slot with a slight rotational play. In the expelling state shown in fig. 3D the spline elements 241 of the clutch element are moved distally out of engagement with the spline elements 204 of the housing thereby allowing rotation of the clutch element relative to the housing. As can be seen from fig. 3D the group of clutch spline elements are now received in the corresponding coupling slot without rotational play.

Fig. 3C shows the clutch element 240 showing the above-described inner circumferential ring-formed array of ratchet teeth 242 and the outer circumferential ring-formed array of axially oriented spline elements 241. As appears, the spline elements are not arranged equidistantly on the ring but in groups, the groups comprising two opposed coupling groups 245 serving as the coupling means engaging the coupling slots 233. Whereas thus only some of the spline elements serve as coupling means between the clutch element and the drive element they all serve as coupling means between the clutch element and the housing splines 204.

Production tolerances on the piston rod, the expelling mechanism, cartridge body, cartridge filling level and other components result in variations in piston rod position and piston position in each device during assembly.

In mechanical devices production, in order to minimize a potential clearance between the piston rod and the piston of the cartridge, positioning may be carried out by initially positioning the piston rod 220 in a nominal position. Due to tolerances various different clearance gaps between the piston and the piston rod will show when the distal and proximal subassemblies of each sample are permanently secured together. On the basis of measurements or estimations, which may be performed at different steps of the assembly process, the actual gap in each sample may be eliminated or at least partly reduced by operating the dose setting and expelling mechanism. Operating the dose setting and expelling mechanism may be carried out either after final assembly or prior to final assembly of the different subassemblies. However such compensation procedure means that the end-of-content mechanism will be operated to a lesser or higher degree even before the device is shipped to the user meaning that the experienced total doseable volume varies from sample to sample. Generally such variations and inconsistencies from one sample to another should be avoided as this may provide the impression that the quality of the device could be somewhat flawed.

Turning to fig. 4 main components of an exemplary first embodiment of an auto-injection device 100 according to the invention is shown. The auto-injection device 100 defines a proximal housing component 101 which accommodates an expelling mechanism comprising a spring-drive. The operating principle of the expelling mechanism may be designed in accordance with the prior art expelling mechanism described in connection with figs. 1 through 3E. However, in other embodiments, alter-native spring-driven expelling mechanisms having other operating principles may be employed. Also manual injection devices configured for manually pressing forward a piston rod during dose expelling may be employed.

A distal housing component 110 holds a cartridge 113 comprising a liquid drug and a piston slideable arranged therein in an axial direction. A cap part 107 is shown. Further a threaded adjustment component 103 is shown.

In the shown embodiment the threaded adjustment component 103 is configured to be axially held at an axial fixed position relative to the proximal housing component 101. Threaded adjustment component 103 is provided with a circumferential bead 103a to be received in a circumferential groove 101a formed in proximal housing component 101.

The threaded adjustment component 103 defines an inner thread 103b. The distal housing component 110 defines an outer thread 110b configured to engage the inner thread 103b.

A plurality of radially inwards protruding axially extending ribs 101c are formed in proximal housing component 101. Ribs 101c are configured for slideably being received in respective longitudinally extending slits 110c formed in distal housing component 110 at the proximal end face. Hence, distal housing component 110 cooperates with the proximal housing component 101 to enable relative axial displacement but prevent relative rotation.

In fig. 5A the above components including subcomponents are shown axially aligned. In fig. 5B, as a first assembly step, the threaded adjustment component 103 is inserted into groove 101a of proximal housing component 101.

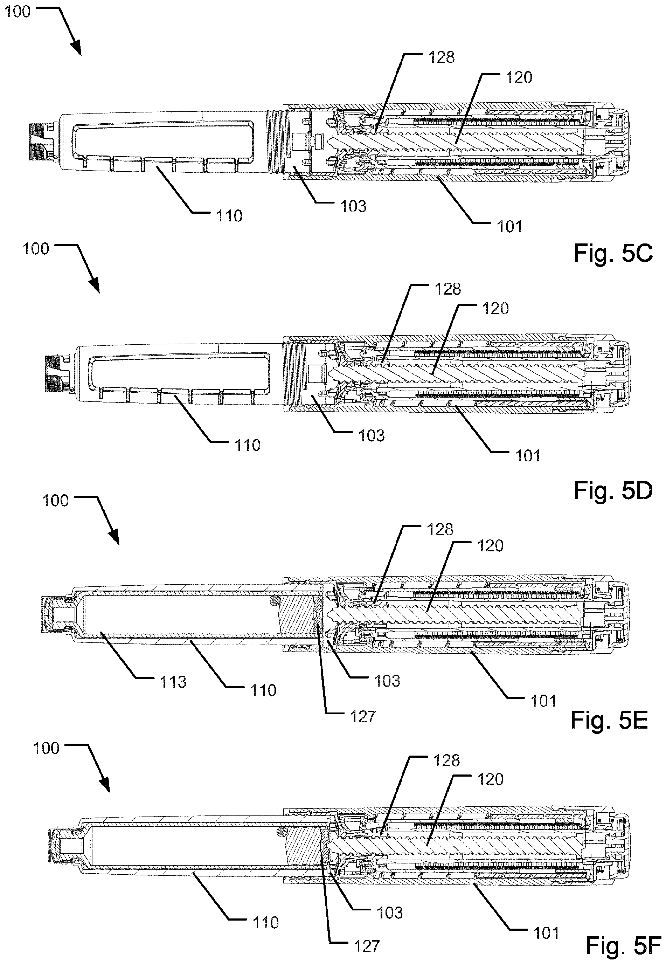

In fig. 5C, the distal housing component 110 has been moved into sliding engagement with the proximal housing component 101. The threads 110b and 103b have not yet been engaged.

Fig 5D shows the distal housing component 110 has been moved further proximally in sliding engagement with the proximal housing component 101. The threads 110b and 103b have not yet been engaged.

In fig. 5E the threads 110b and 103b have only just been engaged. The distal end of the piston rod 120 is situated a distance from the piston washer situated next to the piston of the cartridge 113. By using a not shown tool, the threaded adjustment component 103 may be rotated to cause the distal housing component 110 to be moved further proximally. This has been carried out in fig. 5F where the piston washer has been caused to enter into abutment with the distal end of the piston rod 120. The movement may be performed under control of any type of measurement providing positioning feedback. Suitable methods may include optical measurements, force measurements or similar methods. If a clearance of predetermined magnitude is aimed at, optical measurements may be used or alternatively a force measurement may be used for ensuring abutment with subsequent predetermined axial movement creating the desired clearance.

In some embodiments, the threaded engagement 110b/103b can only be rotated by means of a special tool and cannot be rotated by a human hand without the use of tools. Hence, a proper fastening method can be obtained. Alternatively, or in combination, other fastening methods can be used. For example the movement of the threaded adjustment component 103 may be immobilized by other means such as by means of joining by laser radiation, ultrasonic welding, solvent welding, adhering or similar fastening process either for the threaded adjustment component 103 relative to the distal housing component 110, relative to the proximal housing component 101 or relative to both of the components 110 and 101.

The assembly method has been provided so that the distal housing component 110 and the proximal housing component 101 has a proper rotational orientation relative to each other so that, if required, an intended rotational alignment between a window opening for the scale drum with the inspection openings of the cartridge holder is ensured. The assembled injection device 100 is now ready for labelling and other subsequent finalization steps. It is to be noted that during the assembly method above, the dose setting and expelling mechanism has not been used. Hence, the EOC member 228 has been maintained at the same state throughout the assembly process.

In some embodiments, the above assembly method may include a "standard dose setting and expelling procedure" to be performed either before the states shown in fig. 5A or subsequently. The "standard dose setting and expelling procedure" may be the same across the individual devices of the production line. Such dose setting and expelling procedure may for example be carried out for performing a function check for the dose setting and expelling mechanism and/or for elimination of a previously established fixed "standard gap".

By using the described design and the assembly method, the clearance of each individual pen device can be effectively eliminated or alternatively reduced to a level which is uniform between individual pen devices. In situations where an end-of-content mechanism is incorporated into the device, the pen manufacturing and assembly can be carried out without use of the dose setting and expelling mechanism for evening out the gap between the piston rod and the piston and thus further enables the end-of-content mechanism of each individual device to provide a consistent and predictable total doseable quantity from each individual pen device.

The injection device shown in figs. 1-3 provides a non-limiting example of a pen injector having a design which is suitable for adaptation to the clearance eliminating procedures described herein.

The shown end-of-content limiter provides only a non-limiting example of a suitable end-of-content limiter to be used in connection with the injection device shown in figs. 4 through 5F. Other known end-of-content limiters may alternatively be used in the injection device, such as the end-of-content limiters disclosed in WO 2006/128794 A2, WO 2010/149209 A1, WO 2007/017052 A1, and WO 2013/156224 A1. In accordance with such end-of content mechanisms, instead of an end-of-content limiter formed as a nut member as such, a corresponding track follower that does not necessarily be formed as a "nut" may be used instead, where the track follower may include a first track coupling being coupled to a driver and a second track coupling being coupled to a dose setting element. When used in the present disclosure, the term "nut member" and "end-of-content limiter" will encompass any type of end-of-content limiter.

## Claims

1. A drug delivery device for expelling a dose of drug from a held cartridge, the drug delivery device defining a distal drug outlet end and an opposite proximal end and comprising:
- a distal housing component (110) holding a cartridge (113) comprising a liquid drug and a piston slideable arranged therein in an axial direction,
- a proximal housing component (101) housing at least in part a dose setting and expelling mechanism comprising a piston rod for exerting a force on the piston of the cartridge (113) in a distal direction for expelling a dose,
wherein the distal housing component (110) and the proximal housing component (101) are displaceably arranged in axial direction relative to each other for reducing an axial distance between the piston rod (120) and the piston of the cartridge (113), **characterised in that** a first geometry (110c) of the distal housing component (110) cooperates with a second geometry (101c) of the proximal housing component (101) to enable relative axial displacement but prevent relative rotation,
wherein one of the distal housing component (110) and the proximal housing component (101) comprises a threaded adjustment component (103) being held at an axial fixed location on said one of the distal housing component (110) and the proximal housing component (101) and defining a first thread (103b) being in threaded engagement with a second thread (110b) defined by the other of the distal housing component (110) and the proximal housing component (101),
and
wherein the threaded adjustment component (103) is rotatably fixated so that the adjustment component (103) cannot be rotated without the use of a tool.

2. A drug delivery device as defined in claim 1, wherein the threaded adjustment component (103) is axially held at an axial fixed position relative to the proximal housing component (101) and wherein the distal housing component (110) defines said second thread (110b).

3. A drug delivery device as defined in claim 1, wherein the threaded adjustment component (103) is axially held at an axial fixed position relative to the distal housing component (110) and wherein the proximal housing component (101) defines said second thread.

4. A drug delivery device as defined in any of the claims 1-3, wherein the dose setting and expelling mechanism comprises a dose setting element (250, 260, 270) and a driver (230) configured to drive the piston rod (120) in the distal direction.

5. A drug delivery device as defined in claim 4, wherein the dose setting element (250, 260, 270) rotates relative to the driver (230) during setting of a dose and wherein the dose setting element (250, 260, 270) and the driver (230) rotates together during expelling of a set dose.

6. A drug delivery device as defined in any of the claims 1-5, wherein the drug delivery device further comprises an end-of-content limiter (228) which prevents setting a dose which exceeds a doseable amount of liquid drug remaining in the cartridge (113).

7. A drug delivery device as defined in claim 6, wherein the end-of-content limiter (228) is coupled to the piston rod (120) and the dose setting element (250, 260, 270).

8. A drug delivery device as defined in claim 7, wherein the end-of-content limiter (228) is engaging the piston rod (120) and the dose setting element (250, 260, 270) and wherein the end-of-content limiter (228) moves towards an end-of-content stop geometry provided by one of the piston rod (228) and the dose setting element (250, 260, 270) as the dose setting element is rotated relative to the driver (230) for dialling up a dose.

9. A drug delivery device as defined in claim 6, wherein the end-of-content limiter is coupled to the driver and the dose setting element, the end-of-content limiter being configured to prevent setting of a dose which exceeds a doseable amount of liquid drug remaining in the cartridge.

10. A drug delivery device as defined in claim 9, wherein the end-of-content limiter is engaging the driver and the dose setting element and wherein the end-of-content limiter (228) moves towards an end-of-content stop geometry provided by one of the driver and the dose setting element as the dose setting element is rotated relative to the driver for dialling up a dose.

## Patentansprüche

1. Arzneimittelabgabevorrichtung zum Ausstoßen einer Dosis eines Arzneimittels aus einer gehaltenen Kartusche, wobei die Arzneimittelabgabevorrichtung ein distales Arzneimittel-Auslassende und ein gegenüberliegendes proximales Ende definiert, und umfassend:
- eine distale Gehäusekomponente (110), die eine Kartusche (113) hält, die ein flüssiges Arzneimittel und einen Kolben, der darin in einer axialen Richtung verschiebbar angeordnet ist, umfasst,
- eine proximale Gehäusekomponente (101), in der zumindest teilweise ein Dosiseinstellungs- und Dosisausstoßmechanismus untergebracht ist, der eine Kolbenstange zum Ausüben einer Kraft auf den Kolben der Kartusche (113) in einer distalen Richtung zum Ausstoßen einer Dosis umfasst,
wobei die distale Gehäusekomponente (110) und die proximale Gehäusekomponente (101) in einer axialen Richtung im Verhältnis zueinander verschiebbar angeordnet sind, um eine axiale Entfernung zwischen der Kolbenstange (120) und dem Kolben der Kartusche (113) zu red uzieren,
**dadurch gekennzeichnet, dass** eine erste Geometrie (110c) der distalen Gehäusekomponente (110) mit einer zweiten Geometrie (101c) der proximalen Gehäusekomponente (101) zusammenarbeitet, um eine relative axiale Verschiebung zu ermöglichen, aber eine relative Drehung zu verhindern,
wobei entweder die distale Gehäusekomponente (110) oder die proximale Gehäusekomponente (101) eine mit einem Gewinde versehene Anpassungskomponente (103) umfasst, die an einer axial festen Position an entweder der distalen Gehäusekomponente (110) oder der proximalen Gehäusekomponente (101) gehalten ist und ein erstes Gewinde (103b) definiert, das sich in einem Gewindeeingriff mit einem zweiten Gewinde (110b) befindet, das durch die andere von der distalen Gehäusekomponente (110) und der proximalen Gehäusekomponente (101) definiert wird, und
wobei die mit einem Gewinde versehene Anpassungskomponente (103) so drehfixiert ist, dass die Anpassungskomponente (103) nicht ohne Nutzung eines Werkzeugs gedreht werden kann.

2. Arzneimittelabgabevorrichtung nach Anspruch 1, wobei die mit einem Gewinde versehene Anpassungskomponente (103) axial an einer axial festen Position im Verhältnis zu der proximalen Gehäusekomponente (101) gehalten wird und wobei die distale Gehäusekomponente (110) das zweite Gewinde (110b) definiert.

3. Arzneimittelabgabevorrichtung nach Anspruch 1, wobei die mit einem Gewinde versehene Anpassungskomponente (103) axial an einer axial festen Position im Verhältnis zu der distalen Gehäusekomponente (110) gehalten wird und wobei die proximale Gehäusekomponente (101) das zweite Gewinde definiert.

4. Arzneimittelabgabevorrichtung nach einem der Ansprüche 1-3, wobei der Dosiseinstellungs- und Dosisausstoßmechanismus ein Dosiseinstellungselement (250, 260, 270) und ein Triebelement (230) umfasst, das dazu konfiguriert ist, die Kolbenstange (120) in die distale Richtung zu bewegen.

5. Arzneimittelabgabevorrichtung nach Anspruch 4, wobei sich das Dosiseinstellungselement (250, 260, 270) während des Einstellens einer Dosis im Verhältnis zu dem Triebelement (230) dreht, und wobei sich das Dosiseinstellungselement (250, 260, 270) und das Triebelement (230) während des Ausstoßens einer eingestellten Dosis zusammen drehen.

6. Arzneimittelabgabevorrichtung nach einem der Ansprüche 1-5, wobei die Arzneimittelabgabevorrichtung ferner einen Inhalt-leer-Begrenzer (228) umfasst, der das Einstellen einer Dosis verhindert, die eine dosierbare Menge des flüssigen Arzneimittels übersteigt, die noch in der Kartusche (113) verbleibt.

7. Arzneimittelabgabevorrichtung nach Anspruch 6, wobei der Inhalt-leer-Begrenzer (228) mit der Kolbenstange (120) und dem Dosiseinstellungselement (250, 260, 270) gekoppelt ist.

8. Arzneimittelabgabevorrichtung nach Anspruch 7, wobei der Inhalt-leer-Begrenzer (228) in die Kolbenstange (120) und das Dosiseinstellungselement (250, 260, 270) eingreift und wobei der Inhalt-leer-Begrenzer (228) sich in Richtung einer Inhalt-leer-Halt-Geometrie bewegt, die durch entweder die Kolbenstange (228) oder das Dosiseinstellungselement (250, 260, 270) bereitgestellt wird, während das Dosiseinstellungselement im Verhältnis zum Triebelement (230) für das Wählen einer Dosis gedreht wird.

9. Arzneimittelabgabevorrichtung nach Anspruch 6, wobei der Inhalt-leer-Begrenzer mit dem Triebelement und dem Dosiseinstellungselement gekoppelt ist, wobei der Inhalt-leer-Begrenzer dazu konfiguriert ist, das Einstellen einer Dosis zu verhindern, die eine dosierbare Menge des flüssigen Arzneimittels übersteigt, die noch in der Kartusche verbleibt.

10. Arzneimittelabgabevorrichtung nach Anspruch 9, wobei der Inhalt-leer-Begrenzer in das Triebelement und das Dosiseinstellungselement eingreift und wobei der Inhalt-leer-Begrenzer (228) sich in Richtung einer Inhalt-leer-Halt-Geometrie bewegt, die durch entweder das Triebelement oder das Dosiseinstellungselement bereitgestellt wird, während das Dosiseinstellungselement im Verhältnis zum Triebelement für das Wählen einer Dosis gedreht wird.

## Revendications

1. Dispositif d'administration de médicaments pour l'expulsion d'une dose de médicament à partir d'une cartouche bloquée, le dispositif d'administration de médicament définissant une extrémité distale de sortie de médicament et une extrémité proximale opposée et comprenant :
- un composant de logement distal (110) maintenant une cartouche (113) comprenant un médicament liquide et un piston coulissant qui y est disposé dans une direction axiale,
- un composant de logement proximal (101) logeant au moins en partie un mécanisme de réglage de dose et d'expulsion comprenant une tige de piston pour l'exercice d'une force sur le piston de la cartouche (113) dans une direction distale pour l'expulsion d'une dose,
dans lequel le composant de logement distal (110) et le composant de logement proximal (101) sont disposés de manière déplaçable dans la direction axiale l'un par rapport à l'autre pour la réduction d'une distance axiale entre la tige de piston (120) et le piston de la cartouche (113),
**caractérisé en ce qu'**une première géométrie (110c) du composant de logement distal (110) coopère avec une deuxième géométrie (101c) du composant de logement proximal (101) pour activer un déplacement axial relatif, mais empêcher la rotation relative,
dans lequel l'un du composant de logement distal (110) et du composant de logement proximal (101) comprend un composant de réglage fileté (103) maintenu à un emplacement fixe axial sur ledit un élément parmi le composant de logement distal (110) et le composant de logement proximal (101) et définissant un premier filetage (103b) en engagement fileté avec un deuxième filetage (110b) défini par l'autre du composant de logement distal (110) et du composant de logement proximal (101),
et
dans lequel le composant de réglage fileté (103) est fixé de manière rotative afin que le composant de réglage (103) ne puisse pas être tourné sans l'utilisation d'un outil.

2. Dispositif d'administration de médicament selon la revendication 1, dans lequel le composant de réglage fileté (103) est axialement maintenu dans une position fixe axiale par rapport au composant de logement proximal (101) et dans lequel le composant de logement distal (110) définit ledit deuxième filetage (110b).

3. Dispositif d'administration de médicament selon la revendication 1, dans lequel le composant de réglage fileté (103) est axialement maintenu dans une position fixe axiale par rapport au composant de logement distal (110) et dans lequel le composant de logement proximal (101) définit ledit deuxième filetage.

4. Dispositif d'administration de médicament selon l'une quelconque des revendications 1-3, dans lequel le mécanisme de réglage de dose et d'expulsion comprend un élément de réglage de dose (250, 260, 270) et un élément d'entrainement (230) configuré pour entrainer la tige du piston (120) dans la direction distale.

5. Dispositif d'administration de médicament selon la revendication 4, dans lequel l'élément de réglage de dose (250, 260, 270) pivote par rapport à l'élément d'entrainement (230) durant le réglage d'une dose et dans lequel l'élément de réglage de dose (250, 260, 270) et l'élément d'entrainement (230) pivotent ensemble durant l'expulsion d'une dose définie.

6. Dispositif d'administration de médicament selon l'une quelconque des revendications 1-5, dans lequel le dispositif d'administration de médicament comprend en outre un limiteur de fin de contenu (228) qui empêche le réglage d'une dose qui dépasse une quantité dosable de médicament liquide restant dans la cartouche (113).

7. Dispositif d'administration de médicament selon la revendication 6, dans lequel le limiteur de fin de contenu (228) est couplé à la tige du piston (120) et à l'élément de réglage de dose (250, 260, 270).

8. Dispositif d'administration de médicament selon la revendication 7, dans lequel le limiteur de fin du contenu (228) est en prise avec la tige du piston (120) et l'élément de réglage de dose (250, 260, 270) et dans lequel le limiteur de fin du contenu (228) déplace vers l'avant une géométrie d'arrêt de fin du contenu fournie par l'un parmi la tige de piston (228) et l'élément de réglage de dose (250, 260, 270) lorsque l'élément de réglage de dose pivote par rapport à l'élément d'entraînement (230) pour la composition d'une dose.

9. Dispositif d'administration de médicament selon la revendication 6, dans lequel le limiteur de fin de contenu est couplé à l'élément d'entrainement et à l'élément de réglage de dose, le limiteur de fin de contenu étant configuré pour empêcher le réglage d'une dose qui dépasse une quantité dosable de médicament liquide restant dans la cartouche.

10. Dispositif d'administration de médicament selon la revendication 9, dans lequel le limiteur de fin de contenu est en prise avec l'élément d'entrainement et avec l'élément de réglage de dose et dans lequel le limiteur de fin de contenu (228) déplace vers l'avant une géométrie d'arrêt de fin de contenu fournie par l'un de l'élément d'entrainement et de l'élément de réglage de dose lorsque l'élément de réglage de dose est pivoté par rapport à l'élément d'entrainement pour composer une dose.
